# EUROPEAN PATENT APPLICATION

(11) **EP 2 671 530 A1**
(43) Date of publication of application: **11.12.2013**
(21) Application number: 13169996.9
(22) Date of filing: 31.05.2013
(51) Int. Cl.: A61B 19/02, A61L 2/26

(54) **Surgical instrument tray**

(30) Priority: 04.06.2012 US 201213487792; 16.07.2012 GB 201212610; 15.03.2013 GB 201304712
(71) Applicant: DePuy (Ireland), Ringaskiddy (IE)
(72) Inventor: Birkbeck, Alec, Leeds, West Yorkshire LS11 8DT (GB); Meier, Rusty, Warsaw, IN 46581 (US)
(74) Representative: Alton, Andrew

(57) **Abstract**

A support, a tray for surgical instruments and a method of manufacture are described. The support is used in a surgical tray to support surgical instruments and comprises a rigid part having a free end presenting a formation arranged to accept a part of a surgical instrument and a foot by which the support can be attached to a base of a surgical tray. The support can resiliently flex so as to return the support to an initial state if the support is knocked in use. The surgical tray includes can hold a plurality of surgical instruments, and comprises a body having walls defining an opening and a base, a lid engagable with the opening to close the tray and a plurality of supports extending from the base.

## Description

The present invention relates to containers for surgical instruments and in particular to supports and trays for surgical instruments which can more readily withstand typical use thereof.

Many surgical procedures use a large number of surgical instruments which are typically carried into the operating theatre using a container generally referred to as a tray. Different types of surgical procedures use different types and numbers of instruments. For example, orthopaedic surgical procedures can often use many tens of different instruments. The collection of instruments, and the parts, fixtures and fittings therefore (such as bone pins, screws, *etc.*) are generally referred to as a kit. Hence, the kit of instruments for an orthopaedic surgical procedure, such as a total hip replacement procedure, may involve eight trays of instruments, with each tray holding several instruments and associated accessories.

The instruments in a specific tray can have a wide range of sizes both in terms of their length and width, *i.e.* transverse dimension. However, it is generally preferable to provide trays having a single overall size (in terms of length, width and depth) for ease of manufacture. Usually some form of support structure is provided in a surgical try to support the instruments in certain positions so that they can easily be identified and accessed when needed during a surgical procedure. A tray usually includes instruments needed at a particular stage of a surgical procedure. However, that group of instruments may have widely varying sizes and shapes. As the tray typically has a single fixed depth the support structure needs to be able to accommodate the different sized instruments. Also, all of the instruments need to be retained in the support structure so that they do not move around, fall out or become damaged.

One approach to retaining instruments is to adapt the height of the support structure at different locations within the tray so that a lid of the tray helps to retain the instruments in the support structure during transportation or handling of the tray. For example, a shelf may be provided in the tray with different height portions and/or pockets to accept the different width instruments so that the instruments are all held at a height such that the lid helps to retain all the instruments in their respective supports. However, the inclusion of the shelf increases the weight, cost and complexity of the tray. Further, if instruments or ancillary parts are dropped through the shelf, then it can be difficult and time consuming to retrieve the parts and this can interfere with the efficiency of the surgical workflow.

Another approach is to use plastic supports within a metal tray and to use an O-ring like part to provide a push fit so that the instruments are securely held within the supports such that there is no need for the lid to help retain the instruments within the supports. However, such plastic supports tend to be bulky in order to provide the required mechanical strength and so can be difficult to accommodate within a try at the desired positions.

Another form of secure retention of instruments in a tray is described in US-6,436,357 which describes a sterilizable bracket for supporting medical instruments. The bracket includes a silicone material that surrounds and encapsulates a relatively rigid metal skeleton which is attached to a plurality of studs which secure the bracket to a sterilizable tray. Instruments are received in indentations in the resilient body. The relatively rigid spring tempered skeleton backbone includes a plurality of peaks and valleys oriented in such a way that they align with peaks and indentations of the resilient body respectively. This structure provides additional strength to the resilient silicone body so as to hold the instruments securely in the indentations.

Similarly US-6,382,575 describes an instrument bracket for holding medical instrumentation in a sterilization tray. The bracket includes a clip which is mountable to a base plate of the tray and a holder part. The clip has a U-shaped configuration with spaced upwardly projecting legs accommodating the holder part which is flexible and shape retaining. The holder part includes a recess to accommodate the instrumentation and the clip part compresses and retains the holder part and secures the holder part to the base plate.

However, the corollary of the push fit retention of the instruments is that it is less easy to quickly remove and return the instruments to the tray. Further, the user does not receive the tactile feedback of positive engagement of the instrument back in its correct support structure. Therefore, such trays generally require more care in their use and therefore also impede efficiency of the surgical workflow.

There is therefore a need for a surgical tray which can easily and reliably be used during a surgical procedure to remove and replace instruments and which does not impede the efficiency of the surgical procedure.

A first aspect of the invention provides a support for use in a surgical tray to support surgical instruments, the support comprising: a holder having a free end presenting a formation arranged to accept a part of a surgical instrument; and a foot by which the support can be attached to a base of a surgical tray, and wherein the support can resiliently flex so as to return the support to an initial state if the support is knocked in use.

The supports extend from the base of the tray and so no shelf is needed thereby reducing weight and complexity. The supports can be of differing lengths so that the lid can be used to help retain the instruments in the supports thereby avoiding the need for any further securing mechanism and allowing easy removal and replacement of instruments. The supports can be made long and thin as they can resiliently flex and so if knocked by an instrument during use of the tray, *e*.*g*. a heavy instrument being returned to the tray, then the supports can temporarily deform to help absorb that impact and will also not be permanently deformed or bent out of shape. Otherwise, long thin supports would be prone either to breaking or being permanently bent out of position, thereby preventing the instrument being returned to the tray and requiring the tray to be repaired. Hence, the surgical tray of the invention can be reliably used in a surgical procedure while allowing the rapid removal and return of instruments and so not interfering with the efficiency of the surgical procedure.

Further, by avoiding the need for some other supporting structure, such as a shelf, the weight and complexity of the tray are reduced making them easier to handle and manufacture. Further, there is improved access to the body of the tray for retrieval of parts.

The resilient flex is provided by a part of the support. The resilient flex can be provided by a portion of the holder part and/or the foot part. The resilient flex can be provided by a resilient hinge. The hinge can be a live hinge. The hinge can be provided by a materials property or a mechanical property or a combination thereof. The resilience can be provided by a materials property or a mechanical property or a combination thereof.

The support can include an attachment formation for attaching the support to the base of the tray. The attachment formation can be a push fit or snap-fit formation extending from an underside of the foot. The attachment formation can be arranged to engage with an aperture or opening in the base of the surgical tray.

The attachment formation can include a lower part arranged to pass through and/or under a base of the surgical tray. The holder can include a second end. The second end can be at an opposite end of the holder to the first end. The second end can be received in or extend at least partially or wholly into the lower part of the attachment formation. This can increase the rigidity of the lower part and increase the reliability with which the support is secured to the base by the attachment formation.

The support can have a unitary construction. The support can be made from a single part or piece of material. A property of a part of the material adjacent the foot can differ from a property of a part of the material forming holder part in order to allow the support to resiliently flex. The property can be the stiffness and/or hardness of the material.

The support can have a composite construction. The support can be made from at least two separate parts. The support can include a rigid material. The rigid material can provide a first component including the holder. The support can also include a resiliently flexible material. The resiliently flexible material can provide a second component including the foot.

Hence, the holder provides the tactile feedback of positive engagement to the user that the instrument has been correctly received by the tray supports.

The foot can include a cavity arranged to receive a lower part of the holder therein. The cavity can extend into a lower portion of the attachment mechanism. The cavity can be closed by an end wall. A lower or second end of the holder can seat on or abut the end wall of the cavity.

The rigid material can be a metal. Suitable metals include steel and stainless steel. The resiliently flexible material can be an elastic material, such as a rubber, a rubber-like or an elastomer material. Suitable materials include synthetic rubbers, such as a silicone rubber.

The metal can have a plastic coating. The plastics coating can be nylon. The plastics coating can help to prevent or reduce scratches to the surface of surgical instruments.

The support can include a connector by which the first component and the second component can be attached. A part of the first component and a part of the second component can provide between them the connector. The connector can be a push fit or a snap fit connector.

A further aspect of the invention provides a surgical tray for holding a plurality of surgical instruments. The tray can comprise a body having walls defining a main opening and a base. A lid is engagable with the main opening to close the tray. A plurality of supports according to the preceding aspect of the invention can extend from the base.

Each support can have any of the preferred features of the first aspect of the invention.

The base can have a plurality of apertures or openings. Each support can engage a respective one of the plurality of apertures or openings to attach each of the plurality of supports to the base. The apertures or openings can also assist with the ingress of steam for cleaning and/or sterilisation.

The surgical tray can further comprise a plurality of surgical instruments located within the body of the tray and supported by the plurality of supports.

A further aspect of the invention provides a method of manufacturing a surgical tray. The method can comprise manufacturing a body having walls defining an opening and a base. A lid engagable with the opening to close the tray is also manufactured. A plurality of supports are manufactured. Each of the supports comprises a holder having a free end presenting a formation arranged to accept a part of a surgical instrument and a foot, wherein the support can resiliently flex to return the support to an initial state if the support is knocked in use. The foot of each of the plurality of supports is attached to the base so that the plurality of supports extend from the base. A respective holder can be inserted into a respective foot to secure each support to the base. A connector can be actuated to secure the holder in the foot.

The method can include manufacturing supports or the surgical tray including any of the preferred features of the supports or surgical tray.

An embodiment of the invention will now be described in detail, by way of example only, and with reference to the accompanying drawings, in which:
Figure 1 shows a schematic side elevation view through a surgical tray according to the invention and holding a plurality of instruments;
Figure 2 shows a schematic perspective view of a body of the tray as shown in Figure 1;
Figure 3 shows a schematic perspective view of a support according to the invention for use in the tray of the invention;
Figure 4 shows a partially cut away schematic perspective view of the support shown in Figure 3;
Figure 5 shows a schematic perspective view of a further support also according to the invention and for use in the tray of the invention;
Figure 6 shows a perspective cross sectional view of a part of the further support shown in Figure 5; and
Figure 7 shows a flow chart illustrating a method of manufacture of the support of the invention and the tray of the invention.

Similar items in different Figures share common reference signs unless indicated otherwise.

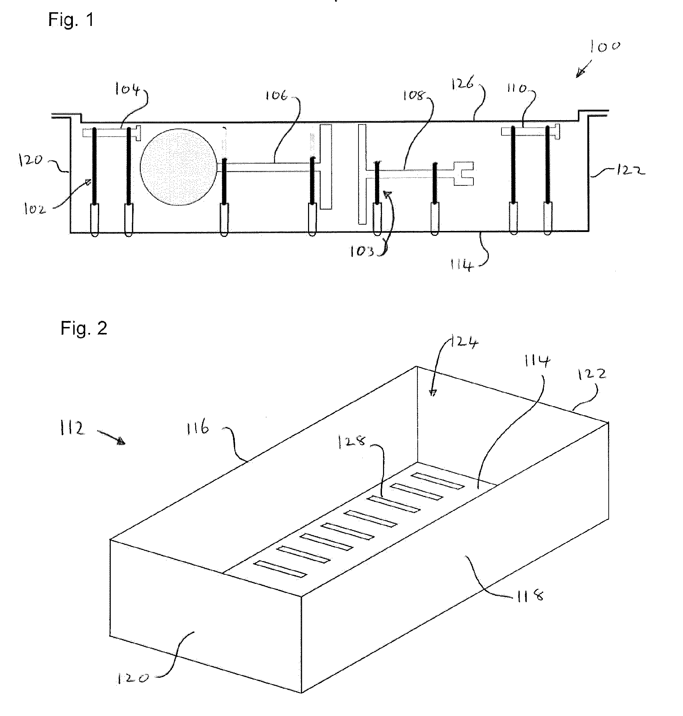

With reference to Figure 1 there is shown a schematic side elevation through a surgical tray 100 according to the invention and including a plurality of supports, *e*.*g*. support 102, also according to the invention. The tray 100 includes a plurality of instruments, 104, 106, 108 110, each of which is supported in the tray by a respective pair of supports. It will be appreciated that each instrument may be supported by fewer or more supports depending on the nature of the instrument and the support. Herein "instrument" is used generally to refer to any item of hardware that may be used in a surgical procedure, including surgical instruments, parts and fittings therefore, and other ancillary components, and is not intended to have a more specific meaning.

Figure 2 shows a schematic perspective view of a body part 112 of the tray 100. The tray has a generally rectangular shape and the body 112 comprises a base 114, a pair of opposed side walls 116, 118 and a pair of opposed end walls 120, 122 and which walls between them define an opening 124 which can be closed by a lid 126. The tray body can be constructed in a number of ways, for example from wire mesh, from perforated metal plates and in other ways generally known in the art. The base 114 includes a plurality of apertures, *e*.*g*. aperture 128, by which the supports are attached to the base to extend in a generally upward direction therefrom. The plurality of base apertures can be provided in a number of configurations, for example as a regular array (as illustrated in Figure 2) which provides a generic tray, in a specific pattern and having a limited number of apertures corresponding to the number of supports to be used, and which provides a use specific tray, or in any variation therebetween, including non-regular arrays and arrangements.

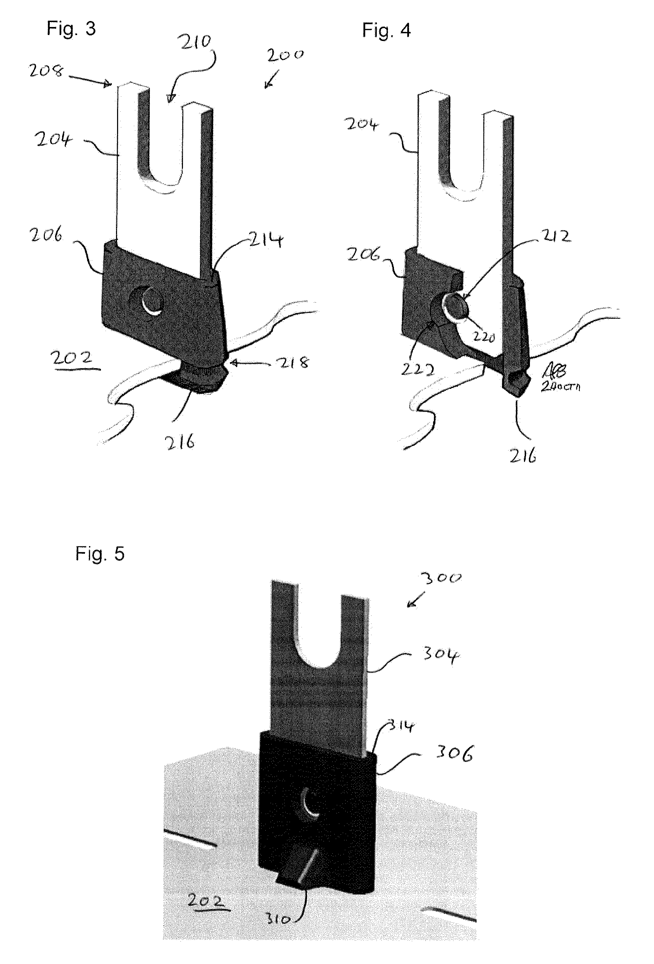

Figure 3 shows a schematic perspective view of a support 200 according to the invention and extending from a portion 202 (shown in cut away form in Figure 3) of the base 114 of the tray. Support 200 has a holder 204 and a foot 206. A free end 208 of the holder 204 presents a formation, in the form of generally U-shaped cut away part or channel 210, to receive and support a part of an instrument in use. The channel 210 has generally parallel side walls at its entrance and a curved bottom and is dimensioned to freely accept, hold and release the part of the surgical instrument it is intended to support. The depth of the channel 210 is sufficient that the instrument is unlikely to become unseated during normal use. The height of the bottom of the channel, relative to the base of the tray is selected such that the instrument will be held at a height within the tray such that the instrument is accommodated within the tray and such that the lid 126 of the tray when in place will act to close off the trough 210 so as to prevent the instrument escaping, as illustrated in Figure 1.

Hence, depending on the instrument to be supported, the depth of the trough and the height of the trough will vary for different supports and depending on what part of the instrument is being supported. For example, as illustrated in Figure 1, support 102 will have a shallow trough at a large height (as instrument 104 has a small width), whereas support 103 will have a deep trough and low height (as instrument 108 has a large width).

The holder 204 has a circular aperture 212 toward a lower end as illustrated in Figure 4 and which forms part of a connector mechanism of the support.

The holder 204 is a rigid part made from a suitable rigid material, preferably a metal, such as steel, and is in the form of a generally rectangular plate approximately 1mm thick. The holder has a plastic coating, e.g. of nylon, approximately 0.1 or 0.2 mm thick in order to help prevent scratching of instruments.

Foot 206 provides resilient flexibility to the overall support so that the support can flex and regain its initial position if knocked by an instrument during use of the tray in surgery. Figure 4 is similar to Figure 3 but shows a partial cut-away view of the foot 206. As well as providing a degree of resilient flexibility to the support 200, foot 206 acts to attach the support to the base. Foot 206 is in the form of a seat or sleeve with a wall 214 defining a central aperture extending along its longitudinal axis and dimensioned to snugly receive a lower part of the holder 204 therein. An attachment part 216 extends from a lower end of the foot and includes a groove 218 extending around it and having a similar width to the thickness of the base 114 of the tray. The groove depth is dimensioned to allow the attachment part 216 to be push fitted into a one of the apertures 128 of the base so as to attach the support 200 to the base 114.

An inner surface of the wall 214 of the foot 206 has a circular protrusion 220 extending therefrom and an opposed part of the wall 214 has a circular aperture 222 therein in registration with the protrusion. The protrusion 220 and aperture 222 provide a foot part of a connector mechanism by which the holder part 204 and foot part 206 can be attached together in a generally push fit manner. The protrusion extends into the aperture 212 in the holder to securely retain the holder in the foot and connect the holder and foot together.

The foot 206 is made from an elastomer, such as a rubber or rubber-like material, for example a silicone rubber, and can be manufactured by injection molding. For example, the foot can be made of an elastomer such as that provided under the name Silplus (RTM) as available from GE Silicones and can have a Shore A hardness in the range of approximately 40 to 80. Hence, the foot 206 provides a semi-rigid seat which is sufficiently rigid to present the free end of the support in an initial position to receive the instrument, but which also has sufficient resilient flex that if impacted, the support will deform but then return back to its initial position without being bent out of shape. As the foot provides the support with the ability to resiliently flex, the holder can be made of a thin plate of stainless steel without the likelihood of the metal becoming permanently bent or otherwise damaged during use of the tray. This is particularly an issue for long supports, such as support 102, compared to shorter supports, such as 103, in which the bending moment for a similar impact will be greater. However, by including in the support a mechanism by which the support can resiliently flex, supports of all different heights that might be required in a single tray can be provided. A range of thicknesses of stainless steel plate can be used depending on the specific details of the application. The thickness can be in the range of approximately 0.6mm to approximately 3.4mm.

Further as no ancillary support structure is needed, such as a shelf, the weight of the tray can be reduced.

Furthermore, as the foot also acts to connect the support to the base, no secondary fixing, such as a rivet, is needed which again reduces weight and also improves ease of manufacture and assembly.

Figure 5 shows a perspective view of a further embodiment of a support 300 according to the invention. Support 300 is generally similar to support 200, and includes a holder 304, but has a different foot 306. The part of the foot 206 above the base 202 shown in Figures 3 and 4 has a generally tapered shape, in which the wall 214 reduces in width moving from the base upward. The combination of the taper, and the materials properties of the silicone rubber, determine the degree of resilience and flex that the foot 206 provides to support 200.

In contrast foot 306 has a generally parallel sides and its wall 314 has a generally constant thickness. Foot 306 has a pair of extensions 310, 312 extending from opposed sides of the foot and generally in the direction in which the support has the greatest degree of flex.

The combination of the extensions, and the materials properties of the silicone rubber, determine the degree of resilience and flex that the foot 306 provides to support 300.

Foot 306 is in the form of a seat or sleeve with a parallel, annular wall 314 of generally constant thickness defining a central aperture 315 extending along its longitudinal axis and having a generally rectangular transverse shape dimensioned to snugly receive the plate-like holder 304 therein. An attachment part 316 extends from a lower end of the foot and includes a groove 318 extending around it and having a similar width to the thickness of the base 114 of the tray. A lower or bottom most end 319 of the attachment part is generally tapered to ease insertion into an aperture 128 in the tray. The groove 318 depth is dimensioned to allow the attachment part 316 to be push fitted into a one of the apertures 128 of the base so as to attach the support to the base 114.

An inner surface of the wall 314 of the foot 306 has a circular protrusion or boss 320 extending therefrom and having a chamfered free end 321. An opposed part of the wall 314 has a circular aperture 322 therein in registration with the protrusion. The protrusion 320 and aperture 322 provide a foot part of a connector mechanism by which the holder and foot part 306 can be attached together in a generally push fit manner. The protrusion extends into a corresponding circular aperture in the holder 304 to securely retain the holder in the foot 306 and connect them together.

The foot 306 is made from an elastomer, such as a rubber or rubber-like material, for example a silicone rubber, and can be manufactured by injection molding. In order to tailor the degree of resilience in different directions, the further foot 306 includes a pair of projections 310, 312 which extend from opposed sides of the foot wall 314 in a direction generally perpendicular to the front 324 and rear 326 faces of the foot wall. The projections extend in a direction generally parallel to the direction in which the foot 306 is likely or intended to deflect if the holder 304 is impacted by an instrument in use. As will be appreciated, the foot 306 will be more resistant to bending or flexing in the perpendicular direction owing to the depth of material of the foot extending in that direction (generally parallel to the front and rear walls of the foot).

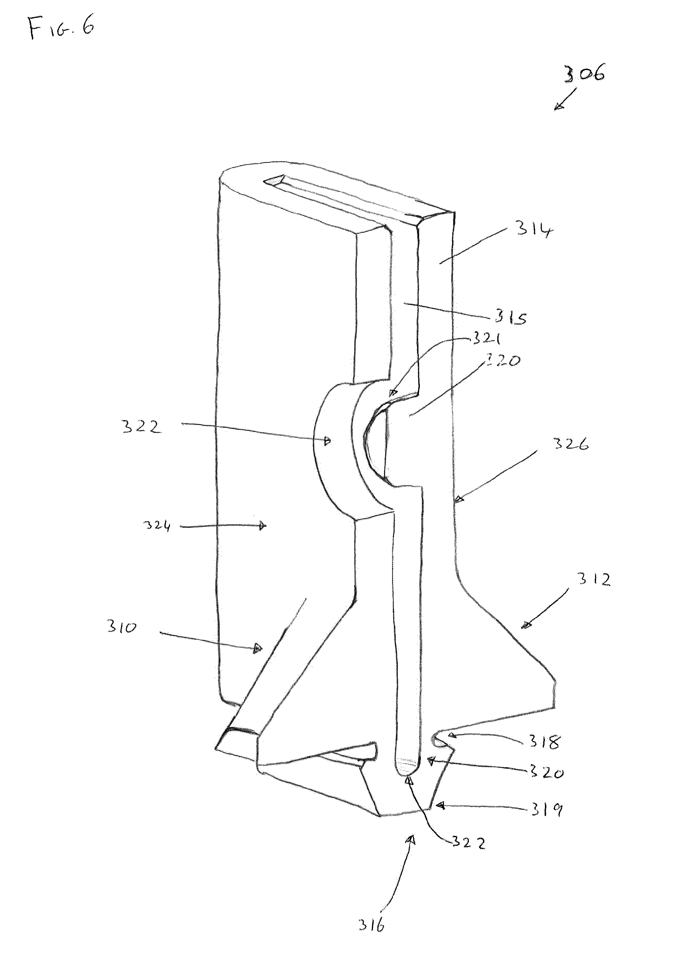

As illustrated in Figures 5 and 6, each extension 310, 312 has a generally wedge-like shape or form and smoothly tapers from a thicker part attached to the wall 314 to a narrower free end part. The degree of taper, length, height and width of the extensions can be selected or the extensions otherwise engineered (for example in terms of their structure or materials properties) so as to provide a desired degree of resilient flex for the overall support in the direction in which they extend.

Hence, the foot 306 provides a semi-rigid seat which is sufficiently rigid to present the free end of the support in an initial position to receive the instrument, but which also has sufficient resilient flex that if impacted, the support will deform but then return back to its initial position without being bent out of shape.

As best illustrated in Figure 6, the central aperture 315 of foot 306 extends into the attachment part 316 which in use extends below the base 114 of the tray. Groove 318 receives the edge of the part of the tray adjacent and defining the tray aperture 128 through which attachment part extends. In particular the lower or bottom most end 319 is located below the lower surface of the base of the tray. Central aperture 315 extends into the lower most portion 319 of the attachment part 316 and in use, as illustrated in Figure 5, a lower most end of holder 304 is located within the lower most part 320 of central aperture 315 and hence extends into the lower most portion 319 of the attachment part 316. This helps to increase the reliability of attachment of the support 300 to the tray base 114.

The foot 306 is initially attached to the tray base without the holder 304 located in the central aperture 315. As the foot 306 is made from a flexible material, it is relatively easy to push the attachment part 316 through the aperture in the tray and engage tray edge parts in groove 318. However, the flexibility and deformability of the attachment part 316 also means that the attachment of the foot to the tray base is not very secure as the attachment part might deform during use, for example if a large bending force were applied to the support, causing the attachment part 316 to escape form the tray aperture 128. However, by inserting holder 304 into the cavity and locating the lower end of holder 314 within the lower most part 320 of central aperture 315 so that a part of the rigid holder is located within the lower most portion 319 of the foot beneath the tray base, the rigidity of the attachment part 316 is increased thereby more securely locking the support 300 to the tray base 114. The best connection can be established by ensuring that the lower most end of holder 304 is seated on the closed end 322 of cavity 315, or put another way with the lower most end of the holder abutting against the end surface 322 of cavity 315.

The connector mechanism 320, 322 also helps to increase the reliability of attachment of the support 300 to the tray base 114 as the connector mechanism acts to retain the lower most end of the support 304 within the lowermost part 319 of the attachment part 316. In the absence of the connector mechanism there is the risk that the holder 304 would start to work its way out of cavity 315 under the action of repeated bending of the support. A reasonably small amount of progression of the holder 304 out of cavity 315 may be sufficient to reduce the rigidity of the attachment part 316 sufficiently for the support to become detached from the tray base. Therefore, the reliability of the connection between the support 300 and tray base 114 can be increased by providing the connector mechanism which acts to securely retain the holder in the foot 306 and prevent movement of the holder 3 04 along the cavity 315.

Further, the resilient material of the foot 306 helps to provide a natural locking of the connector mechanism as the resilient material urges boss 320 through an aperture in the holder 304 and toward and into aperture 322.

It will be appreciated that holder 200 is arranged similarly, as best illustrated in Figure 4

Hence it will be appreciated that there are a wide number of ways in which the support can be engineered in order to provide desired degrees of resilience and of flex and in what directions. Properties that may be varied include generally materials properties and/or mechanical properties and/or shape properties. For example, in some embodiments, the foot may not be made of a resilient material, but instead may be made from a rigid material but which includes a resilient part, such as a spring (for example a torsion spring), which provides the resilience and ability to flex. In some embodiments, the support may be made from a single part (rather than two separate parts) and the material or mechanical properties of a unitary support may be varied in order to provide the rigidity of the holder and the resilient flexibility. For example, the support may be made from a single piece or part, but the free end providing the holder may be treated (for example by implantation or doping with another material) in order to be more rigid and the foot part may not be so treated, or treated differently in order to be resilient and flexible. Therefore, various types of support are envisaged including both the compound type illustrated in Figures 3 to 6 (made from multiple parts attached together) and a unitary type (made from a single part providing both the foot and holder) as described above and including a mechanical hinge or living hinge to provide flexibility.

It will be appreciated that the support of the invention provides a more reliable tray as it is more able to withstand typical use in an operating theatre in which instruments are rapidly taken from and replaced in a tray. As the retaining formation 210 in the free end of the support freely retains the instrument (*i.e.* there is no push fit, clamping or other mechanism holding the instrument in the supports) the instruments can be quickly removed from the tray and replaced on the supports. Further, as the free ends of the supports are rigid, tactile feedback of "hard-on-hard" placement makes it easier for users to be confident that the instrument has been correctly replaced in the tray. Furthermore, the user can be less careful in returning instruments to the tray as the supports can flex and automatically return to their initial position if knocked by an instrument thereby reducing the chance of damage to the instrument or supports. Yet further, the resilience of the foot provides a certain amount of damping to prevent inertial damage to the supports and/or instruments during rough handling or mishandling of the tray (*e.g.* a tray being dropped). Many of these benefits arise from a simpler tray construction which is also light weight and improves the ease of manufacture of the tray.

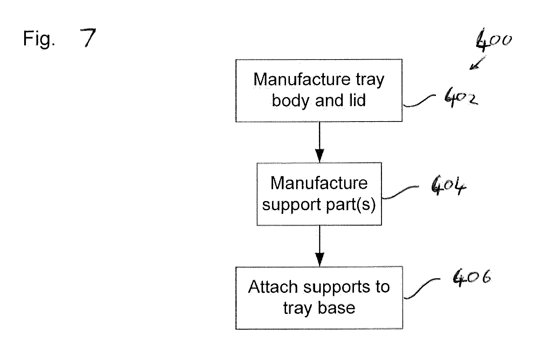

A method of manufacture 400 of the tray 100 also according to the invention will now be described with reference to Figure 7. At step 402, the body of the tray and the lid are manufactured. As described above the body and the lid are typically made of metal and can be in the form of sheets or plates of metal or wire mesh either bent, folded or otherwise formed and joined together using suitable fastenings, such as rivets. As described above, the base part of the tray body includes a plurality of apertures which may be punched, machined, cut or otherwise formed in the base of the tray. The number, pattern and arrangement of apertures can depend on whether the tray is a generic tray or a specialised tray.

At 404 a plurality of supports are made. If the supports have a unitary construction then they will generally have a one part form. Otherwise, if they have a compound construction, then they will have a multi-part form. For example, the supports 200, 300, can be manufactured by cutting a plurality of holders from thin sheets of steel and then plastic coating them and the foot parts can be formed by injection molding. The rigid holder parts 204, 304 can be inserted into the foot parts and the connector parts of the holder part and foot part co-operate to securely connect them together. It will be appreciated that the order of steps 402 and 404 is irrelevant and the steps can be carried out in parallel as well.

Once the supports and tray body have been manufactured, then at step 406, the supports are attached to the base of the tray. As described above, the foot parts 206, 306, include an attachment formation on their lower end which is push fit to engage with respective apertures in the base of the tray. After push fitting an attachment formation 216, 316 into a tray aperture, the holder part 204, 304 is pushed into the cavity 315, until its lower most end abuts against the closed end 322 of the cavity so as to increase the rigidity of the attachment formation below the tray base and more securely attach the support to the tray base. The connector mechanism is also engaged with the holder 204, 304 to prevent the holder 204, 304 from working lose from the foot 206, 306.

The specific height, depth and shape of the retaining formations in the free ends of the rigid parts will vary depending on the instrument, and part of the instrument, that each support is intended to support. It may be that a suite of four or five generic plates 204 may be sufficient to support all or most instruments, with more specific plate shapes being manufactured on an *ad hoc* basis. The position of attachment within the base of the tray will also vary depending on the specific collection of instruments to be provided in an individual tray. Once the supports have been attached, the tray is ready to receive the instruments and the lid of the tray can then be attached so as to help retain the instruments within the tray.

Although specific embodiments of the invention have been described above, it will be appreciated that a large number of variations, modifications and changes are possible and will be apparent to a person of ordinary skill in the art from the teaching herein.

## Claims

1. A support for use in a surgical tray to support one or more surgical instruments, the surgical tray comprising a base having a plurality of openings formed therein, the support comprising:
a holder made from a rigid material having a first end having a formation arranged to accept a part of a surgical instrument and a second end; and
a foot made from a resiliently flexible material and connected to the holder, the foot having a first portion configured to be attached to the base of the surgical tray, the first portion including a lower part arranged to pass through and under the base of the surgical tray and wherein the second end of the holder extends into the lower part of the first portion to secure the foot to the base of the surgical tray and the support resiliently flexes to return the holder to an initial state if the holder is knocked in use.

2. A support as claimed in claim 1, wherein the first portion includes a push fit or snap-fit formation extending from an underside of the foot and arranged to engage with a one of the openings in the base of the surgical tray.

3. A support as claimed in claims 1 or 2, wherein the foot has a cavity extending into the lower part of the first portion and the holder is received in the cavity.

4. A support as claimed in claims 1 or 2, wherein the support has a composite construction.

5. A support as claimed in claim 3, wherein the cavity has a closed end surface and the holder has a lower end surface which is seated on the closed end surface of the cavity.

6. A support as claimed in claim 1, wherein the rigid material is a metal and the resiliently flexible material is a synthetic rubber.

7. A support as claimed in claim 6, wherein the metal has a plastics coating.

8. A surgical tray as claimed in claim 1, wherein the support includes a connector by which the holder and the foot can be attached to secure the holder within the foot.

9. A support as claimed in claim 8, wherein a part of the holder and a part of the second component provide between them the connector and wherein the connector is a push fit or a snap fit connector.

10. A surgical tray for holding a plurality of surgical instruments, the tray comprising:
a body having walls defining a main opening and a base having a plurality of openings;
a lid engagable with the main opening to close the tray; and
a plurality of supports as claimed in any preceding claim extending from the base.

11. A surgical tray as claimed in claim 10, wherein each support engages a respective one of the plurality of openings to attach each of the plurality of supports to the base.

12. A surgical tray as claimed in claim 10 or 11 and further comprising:
a plurality of surgical instruments located within the body of the tray and supported by the plurality of supports.

13. A method of manufacturing a surgical tray, comprising:
manufacturing a body having walls defining an opening and a base;
manufacturing a lid engagable with the opening to close the tray;
manufacturing a plurality of supports, each of the plurality of supports comprising:
a holder made from a rigid material having a first end presenting a formation arranged to accept a part of a surgical instrument and a second end; and
a foot made from a resiliently flexible material, wherein the support can resiliently flex to return the support to an initial state if the support is knocked in use; and
attaching a respective foot of each of the plurality of supports to the base and inserting a respective holder into each foot so that the plurality of supports are securely attached to and extend from the base.
